# EUROPEAN PATENT APPLICATION

(11) **EP 4 000 512 A1**
(43) Date of publication of application: **25.05.2022**
(21) Application number: 20208435.6
(22) Date of filing: 18.11.2020
(51) Int. Cl.: A61B 5/11, A61B 5/145, A61B 5/00

(54) **SYSTEMS AND METHODS FOR PROVIDING EXERCISE ADVICE**

(71) Applicant: Koninklijke Philips N.V., 5656 AG Eindhoven (NL)
(72) Inventor: DELLIMORE, Kiran Hamilton J., 5656 AE Eindhoven (NL); BONOMI, Alberto Giovanni, 5656 AE Eindhoven (NL); HUIJBREGTS, Laurentia Johanna, 5656 AE Eindhoven (NL); JOHNSON, Mark Thomas, 5656 AE Eindhoven (NL)
(74) Representative: Philips Intellectual Property & Standards

(57) **Abstract**

A system for providing exercise advice comprises a sensor for a sweat parameter, a controller and an output device. The controller is configured to receive (92) the sweat parameter, to derive (94) from the sweat parameter a sweat rate at which sweat is excreted by a wearer, and to derive (96) from the sweat rate an exercise load. The controller is further configured to control the output device to provide (98) information to the wearer relating to the derived exercise load.

## Description

### FIELD OF THE INVENTION

This invention relates to systems and methods for providing exercise advice. The systems and method can, for example, be used to help people suffering from heart failure to exercise in a safe and healthy way.

### BACKGROUND OF THE INVENTION

In order to slow the progression of heart failure and support a vital lifestyle, heart failure patients need to maintain a certain level of physical activity by performing exercises. However, these exercises need to be conducted with great caution, since at all times the heart rate has to be kept within safe limits defined by the doctor. However, in many cases the heart rate cannot be reliably measured due to arrhythmias (i.e., irregular heartbeats), which are very common among heart failure patients. It is believed that between 30-90% of heart failure patients suffer from arrhythmias. Furthermore, cardiac patients are very often treated with rate control medication (such as beta blockers, calcium channel blockers, sodium channel blockers, potassium channel blockers, etc.) that reduce the overall heart rate and heart rate response to physical activity making it very challenging for doctors and physical therapists to determine the optimal training regimen for a patient, especially when trying to account for heart rate influencing factors such as disease progression, medication adherence, and training effect.

In light of the above, many heart failure patients lack confidence when performing therapeutic training exercises or physically strenuous activities, which causes them to feel out of breath or elevate their heart rate since they fear either death or experiencing a cardiac decompensation or myocardial infarction. As a result, heart failure patients tend to avoid doing exercise, which, in turn, leads to worsening of their physical condition thereby increasing their risk of a decompensation event or myocardial infarction.

Due to the high occurrence of arrhythmias in heart failure patients and the widespread use of rate control medication in cardiac patients, conventional heart rate fencing approaches utilizing, e.g., wearable or exercise machine based heart rate monitors, are unreliable and potentially dangerous for heart failure patients.

It is known that an alternative approach to limiting the heart rate is to monitor energy expenditure. Whilst it is possible to monitor such patients in a laboratory setting while wearing an indirect calorimeter measuring breath-by-breath oxygen and carbon dioxide production, this approach remains impractical for domestic and outdoor applications.

CN 108836398 A discloses a device for assessing the fitness of a person based on the amount of sodium excreted with the sweat during exercise. This device may be suitable for an assessment of the overall fitness of a person based on an exercise session. The device though only addresses the question of fitness after the exercise. It does not enable any conclusions to be drawn about the actual exercise load a person is experiencing at a given time during the exercise.

There remains a need for systems and methods that can help a person and in particular a person suffering from heart failure to safely and effectively exercise outside of a laboratory setting.

### SUMMARY OF THE INVENTION

The invention is defined by the independent claims. The dependent claims define advantageous embodiments.

According to examples in accordance with an aspect of the invention, there is provided a system for providing exercise advice comprising:
a wearable sensor for measuring a sweat parameter;
a controller; and
an output device,
wherein the controller is configured to:
receive the sweat parameter;
derive from the sweat parameter a sweat rate at which sweat is excreted by a wearer;
derive from the sweat rate an exercise load; and
control the output device to provide information to the wearer relating to the derived exercise load.

People suffering from heart failure are advised to keep a certain level of physical fitness by performing exercise. However, such exercise must be performed with great caution, since at all times the exercise load has to be kept within safe limits defined by the doctor. Otherwise, further damage to the heart can occur. In many cases though it is not possible to reliably measure the heart rate of a person due to the presence of arrhythmias. Furthermore, people suffering from heart failure are very often treated with heart rate control medication and this reduces the overall heart rate and the heart rate response to physical activity making it very challenging for doctors and physical therapists to determine the optimal training regimen for a person, especially when trying to account for heart rate influencing factors such as disease progression, medication adherence, and training effect.

The rate at which a person sweats can function as an indicator for the exercise intensity. The system can be used to help a patient to stay within healthy levels of exercise without having to rely on heart rate monitoring.

The information relating to the derived exercise load may be selected from advice to increase the intensity of the exercise, advice to decrease the intensity of the exercise, advice on the length of time the exercise should be continued for and a notification that the exercise needs to be stopped immediately.

In one set of examples, the wearable sensor comprises a sensor for measuring a total sweat volume or sweat rate, for example by mass or conductivity measurements. The system may then further comprise a sensor for at least one biomarker present in the sweat of the wearer, wherein the controller is configured to derive the exercise load further taking account of any of said at least one biomarker which have been sensed. Thus, in this case, the primary sweat rate measurement is based on a total volume or mass measurement, and the biomarker sensing provides additional data, for example data relating to the fitness level or the hydration of the user.

Biomarkers present in the sweat of a person can also provide further information on the level of exercise making the overall determination of the exercise level more reliable.

In another set of examples, the wearable sensor is itself a sensor for at least one biomarker present in the sweat of the wearer. The biomarker concentration is for example correlated with the sweat rate (or a surrogate for the sweat rate) and with the exercise load, so even a biomarker sensor may function as the primary sweat parameter sensor used to derive the sweat rate.

Furthermore, the system may, for example, further comprise a position and/or motion sensor, wherein the controller is configured to derive the exercise load further taking account of the output of the position and/or motion sensor.

The use of position sensors and/or GPS sensors can provide further information on the nature of the exercise performed and help contextualise the data obtained.

In addition, the system may further comprise an environment sensor for measuring at least one environmental condition, wherein the controller is configured to derive the exercise load further taking account of the output of the environment sensor.

The use of environmental sensors can increase the accuracy of the correlation between the sweat rate and the exercise level by making it possible to take account of other factors influencing the sweat rate such as temperature and humidity.

Furthermore, the system may further comprise a communications interface for receiving information from an external source to be used in deriving the exercise load.

A communications interface can be used to receive information such as weather information or other information relating to the environmental conditions from outside sources.

In some instances, the controller may be further configured to determine a rate of change of the sweat rate and to derive the exercise load further taking account of the rate of change of the sweat rate.

Determining the rate of change in the sweat rate can help to discern between exercise related changes in the sweat rate which tend to happen over longer timescales of minutes and, for example, changes in the sweat rate due to emotional reasons which happen on a shorter timescale of seconds. Due to their rapid response to changes in the amount of sweat present, galvanic skin response sensors (GSR) can be particularly useful to discern exercise related changes in the sweat rate from changes in the sweat rate due to, e.g., emotional reasons.

The controller may also be configured to provide post-exercise advice.

The post-exercise advice may thereby be selected from advice on the intensity of the next exercise, advice on the duration of the next exercise, advice on the time to wait until the next exercise and combinations thereof.

Post-exercise advice can further help a patient plan and balance their exercise load to avoid further damage to the heart.

The invention also provides a method for providing exercise advice to a person comprising the steps of:
measuring a sweat parameter of the person;
deriving from the sweat parameter a sweat rate at which sweat is excreted by the person;
deriving an exercise load from the sweat rate; and
providing information to the person relating to the derived exercise load.

The information relating to the derived exercise load may be selected from advice to increase the intensity of the exercise, advice to decrease the intensity of the exercise, advice on the length of time the exercise should be continued for and a notification that the exercise needs to be stopped immediately.

The present method can provide exercise advice in situations where other, conventional methods such as heart rate monitoring are not a suitable or reliable way to determine exercise intensity and is particularly useful for people suffering from heart failure.

The method may further comprise determining a rate of change in the sweat rate, and determining the exercise load further taking account of the rate of change.

Determining the rate of change in the sweat rate can help to discern between exercise related changes in the sweat rate which tend to happen over longer timescales of minutes and, for example, changes in the sweat rate due to emotional reasons which happen on a shorter timescale of seconds.

Measuring a sweat parameter (from which the sweat rate or a surrogate signal representing the actual sweat rate is derived) may comprise:
measuring a total sweat volume or sweat rate; or
sensing at least one biomarker present in the sweat of the wearer; or
measuring a total sweat volume or sweat rate and sensing at least one biomarker present in the sweat of the wearer, wherein deriving an exercise load takes account of said the total sweat volume or sweat rate and the at least one biomarker.

In addition, the method may comprise measuring environmental conditions around the person and/or sensing a position and/or motion of the person and deriving the exercise load further taking account of the measured environmental conditions and/or the movement of the person.

Measuring or receiving environmental conditions can increase the accuracy of the correlation between the sweat rate and the exercise level by making it possible to take account of other factors influencing the sweat rate such as temperature and humidity.

Sensing the position and/or motion of a person can provide further information on the nature of the exercise performed and help contextualize the data obtained.

The method may further comprise providing post-exercise advice.

The post-exercise advice may be selected from advice on the intensity of the next exercise, advice on the duration of the next exercise, advice on the time to wait until the next exercise and combinations thereof.

Post-exercise advice can further help a person plan and balance their exercise load for example to avoid heart damage in a person suffering from heart failure.

The invention also provides a computer program comprising computer program code means which is adapted, when said program is run on a computer, to implement the above method for providing exercise advice.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a first example of a system for providing exercise advice according to the invention;
Figure 2 shows a graph showing the variation of a sweat property of a subject over time;
Figure 3 shows a table showing the concentration of sodium ions and potassium ions in the sweat of a subject dependent on the exercise intensity;
Figure 4 shows a graph plotting the lactate concentration over time during an exercise session;
Figure 5 shows a graph plotting the sweat lactate concentration versus the sweat rate;
Figure 6 shows a graph showing the correlation between the local sweat rate on the left forearm and the whole body sweat rate of a subject;
Figure 7 shows a second example of a system for providing exercise advice according to the invention;
Figure 8 shows a first graph of the development of the sweat rate over time during exercise;
Figure 9 shows a second graph of the development of the sweat rate over time during exercise;
Figure 10 shows a flow diagram for a first example of a method for providing exercise advice according to the invention; and
Figure 11 shows a flow diagram for a second example of a method for providing exercise advice according to the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides systems and methods for providing exercise advice based on the sweat rate of a person. The system comprises a wearable sensor for measuring a sweat parameter, a controller and an output device. The controller is configured to receive the sweat parameter and to derive the rate at which sweat is excreted by a wearer. The controller is further configured to derive an exercise load from the sweat rate. The controller is configured to control the output device to provide information to the wearer relating to the derived exercise load. The information relating to the derived exercise load may be selected from advice to increase the intensity of the exercise, advice to decrease the intensity of the exercise, advice on the length of the exercise and a notification that the exercise needs to be stopped immediately.

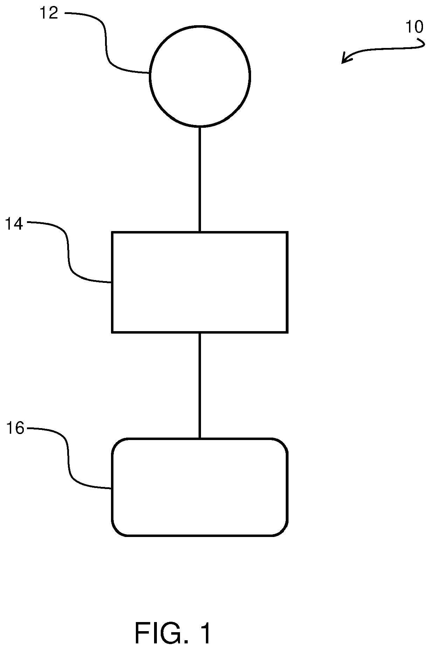

Figure 1 shows an example of a system 10 for providing exercise advice according to the invention.

The system 10 comprises a sensor 12 for measuring a sweat parameter, a controller 14 and an output device 16.

The sensor 12 for measuring a sweat parameter can be any sensor that is capable of sensing a parameter indicative of the sweat rate of a person. Examples of sensors that can be employed as the sensor for a sweat parameter 12 include GSR sensors, calorimetric flow sensors, microbalances or resonance based sensors. MEMS calorimetric flow sensors measure the flow rate by measuring the asymmetry of the temperature profile modulated by the flow of sweat through e.g., a microchannel. Microbalances/resonance based sensors (e.g., a quartz crystal microbalance) measure a mass variation per unit area by measuring the change in frequency of a quartz crystal resonator.

The above sensing approaches all measure a sweat volume or a sweat rate, by making a measurement of the sweat produced. In addition, it is also possible to measure the sweat rate for example based on the amount of certain biomarkers excreted with the sweat. Biomarkers suitable for the determination of the sweat rate include e.g. Na⁺, K⁺, Cl⁻ and lactate.

GSR sensors have a particularly rapid response to changes in the sweat rate and may be useful to discern short timescale changes in the sweat rate that might not be exercise related from longer timescale changes in the sweat rate caused by exercise. Short timescale changes in the sweat rate may, for example, be caused by emotional reasons.

The sensor 12 is designed to be worn on the skin of a user. The sensor can thereby be integrated into a body worn device, such as a smart or fitness watch, or be a separate unit in the form of a patch or attached to or integrated into a body worn strap.

The sensor 12 is in communication with the controller 14. The communication can be wired or wireless, such as a Bluetooth or NFC connection.

The system 10 of Figure 1 only comprises one sensor 12 but it is also possible for a system to comprise multiple such sensors, for example, sensors that can be worn on different parts of the body.

The controller 14 receives the sweat parameter from the sensor 12. The controller 14 is configured to derive a sweat rate at which sweat is excreted by the wearer. The controller 14 is further configured to derive the exercise load from the sweat rate. The determination of the exercise load from the sweat rate can be based on general, for example empirically derived correlations. Alternatively, this determination can be made based on calculations which are specific for the wearer. These can, for example be derived from one or more initial supervised exercise sessions. It is also possible to use combinations of general empirical calculations and calculations taking into account parameters specific to the wearer. The controller 14 can also employ machine learning to adapt the determination of the exercise load to changes in the individual situation of the wearer over time.

The controller 14 is further configured to control the output device 16 to provide information to the wearer relating to the derived exercise load. The information may be selected from advice to increase the intensity of the exercise, advice to decrease the intensity of the exercise, advice on the length of time the exercise should be continued for and a notification that the exercise needs to be stopped immediately.

The output device 16 can provide the information relating to the derived exercise load to the wearer in a suitable manner. Suitable means of conveying this information include, for example, visual, aural or haptic means as well as combinations thereof. Accordingly, the output device 16 can be a display, such as the display of a smart or fitness watch or a smartphone, a loudspeaker or a set of headphones or any device that can provide haptic feedback such as a vibrating bracelet or patch.

The communication between the controller 14 and the output device 16 can be wired or wireless, such as a Bluetooth or NFC connection.

The components of the system 10 can be in the form of individual components or combined into one or two combined devices. For example, the sensor 12, the controller 14 and the output device 16 can be integrated into one, for example wrist mounted, device. In another example the sensor 12 and the controller 14 can be combined in a body worn patch and the output device can be a separate unit. In a further example, the sensor 12 can be a body worn unit and the controller 14 and the output unit 16 can be integrated into one unit. Any parts of the system 10 can also be integrated into other devices such as smart or fitness watches or smart phones as long as they can still fulfil their intended function.

It has been shown that the sweat rate is linearly correlated with exercise intensity as determined in clinical laboratory setting while wearing an energy expenditure meter (indirect calorimeter). In general, lower exercise intensity is correlated with lower sweat rate while higher exercise intensity corresponds with higher sweat rate. Moreover, it has also been demonstrated that a locally measured sweat rate is highly correlated with whole body sweat rate. This indicates that the measurement of the sweat rate at a single body location (e.g., the forearm or forehead) provides a reliable measure of the overall sweat rate of an individual. This sweat rate in turn can be correlated to exercise intensity, taking into account, where necessary other factors such as environmental temperature and humidity.

The rate at which a person sweats can therefore function as an indicator for the exercise intensity. The system can therefore be used to help a patient to stay within healthy levels of exercise without having to rely on heart rate monitoring.

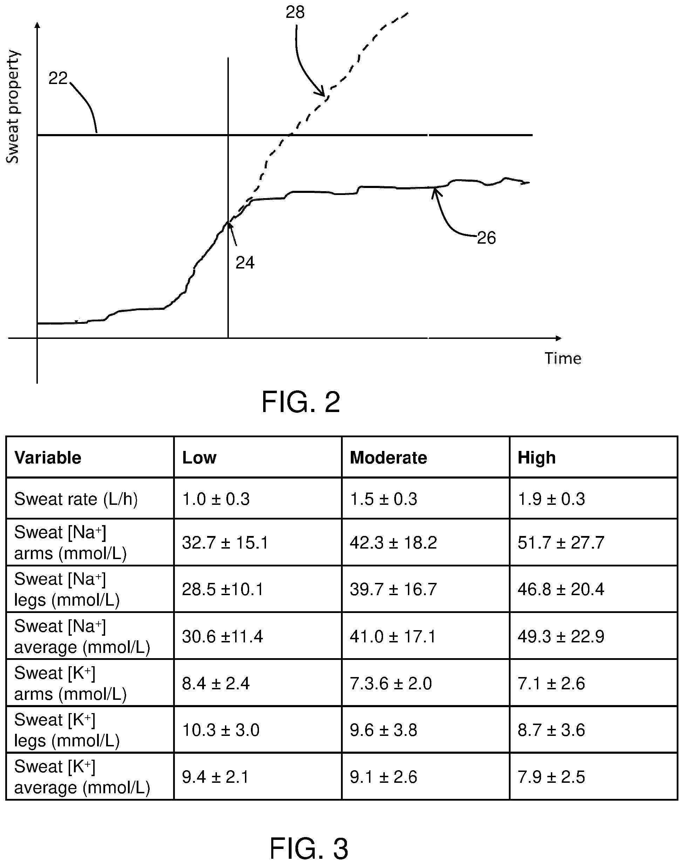

Figure 2 is a graph to illustrate the method implemented by the system of Figure 1.

The graph shows the variation of a sweat property of a subject over time. At time 20, the subject starts to exercise. The sweat property increased over time, and hence approached a safety limit 22 that applies to that particular subject. The safety limit 22 can be either determined based on empirical observations or determined for each individual subject for example based on graded exercise under close supervision by a doctor or a physiotherapist. The safety limit 22 depends e.g. on the individual patient and the environmental conditions. It could, for example, be 5 to 10 times the sweat rate at rest. Assuming a sweat rate of 0.1 mg/(cm²•min) at rest, then the safety limit 22 could be prescribed between say 0.5 to 1.0 mg/(cm²•min).

At time 24, the subject is given a warning by the system to reduce the exercise rate in order to prevent crossing the safety zone limit. The time 24 is a time before the subject has crossed a threshold of energy expenditure that would be unsafe or dangerous, i.e. the safety limit 22. The time 24 should be sufficient for the subject to be able to adapt their exercise intensity so that they do not cross the safety limit 22. The time 24 can, for example, be defined as a point of time at which the sweat rate is 40-60% of the sweat rate at the safety limit 22. Plot 26 shows the sweat property if the subject does reduce the exercise rate and plot 28 shows the sweat property if the subject continues to exercise at the same rate.

Figure 3 shows some examples of measured values of various sweat parameters for different exercise levels (low, moderate, high). The sweat rate is shown, as well as the concentration of Na⁺ and K⁺ ions at different body parts (arms, legs, and the average between the two).

Figure 3 shows that the concentration of Na⁺ and K⁺ in sweat, is linearly or quasi-linearly correlated with exercise intensity. In general, lower exercise intensity is correlated with lower Na⁺ and higher K⁺ concentrations, and vice versa, as seen in Figure 3.

In the case of lactate, higher lactate concentrations are linked to lower sweat rate, while lower lactate concentrations correspond with high exercise intensity.

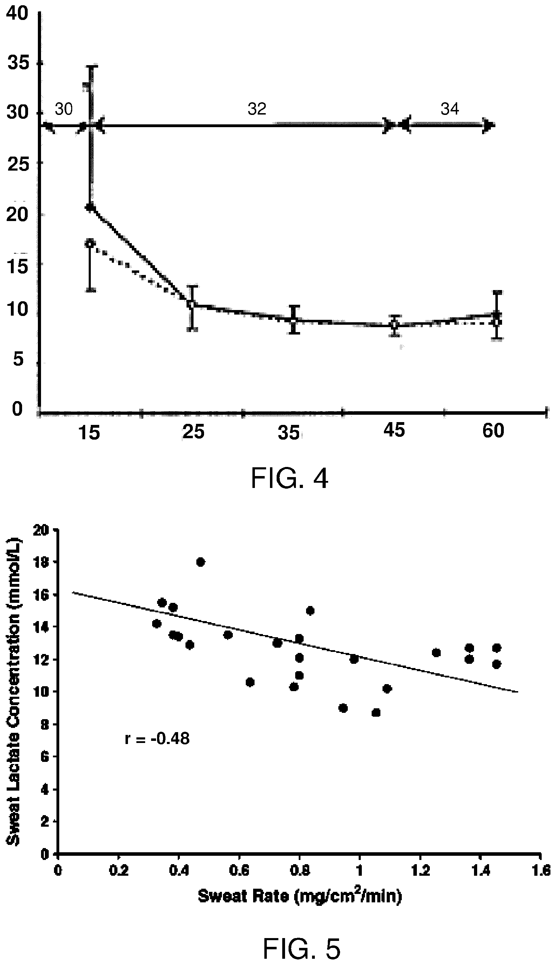

Figure 4 shows a plot of lactate concentration (mmol/L, y-axis) versus time (mins, x-axis) during warm up 30, exercise 32 and rest 34. The solid plot shows continuous exercise and the dotted plot shows intermittent exercise.

Physiologically, this inverse relationship occurs due to the dilution of sweat lactate by the increased volume of sweat produced during intense exercise. Although the transport of lactate from plasma to sweat is still poorly understood, it is clear that sweat lactate is at least an indirect indicator of physical exertion and is likely directly related to the exertion of the sweat glands themselves in response to whole body exertion.

This inverse relationship between the sweat rate and the sweat lactate concentration can also be seen in Figure 5, which shows a graph plotting the sweat lactate concentration (mmol/L, y-axis) versus the sweat rate (mg/(cm²•min), x-axis). Figure 5 clearly shows that the sweat lactate concentration decreases with an increase in the sweat rate.

It has been estimated that roughly 90-95% of lactate in sweat is produced by the sweat glands themselves due to the active role that they play in thermoregulation of the human body during exercise. This makes lactate another useful biomarker to measure when assessing the intensity of physical activity.

It can be seen that the concentration levels of various biomarkers thus vary (in different ways) with sweat rate and with exercise load. This correlation in turn can be used to determine the sweat rate based on the concentration of such biomarkers. Similarly, the skin conductivity measured by a GSR sensor (over time) also correlates with sweat rate.

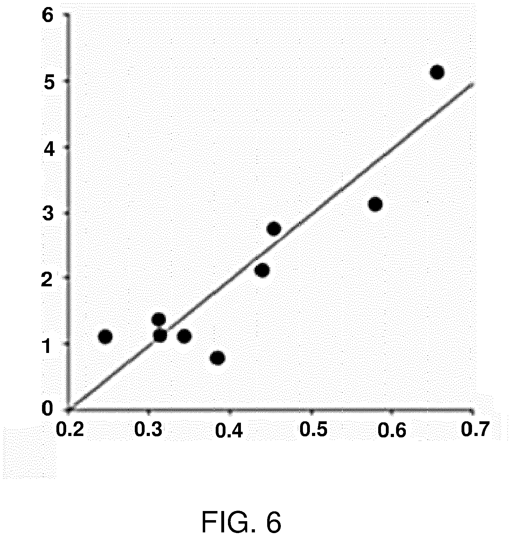

Figure 6 shows the correlation between a whole body sweat rate (mg/(cm²•min), x-axis) and a local (left forearm) sweat rate (mg/(cm²•min), y-axis), during graded exercise until exhaustion. This correlation also applies to sweat measurements taken on other parts of the body. In general, it is therefore possibly to draw conclusions about the total body sweat rate from measurements taken at any part of the body.

The sweat parameter being measured may thus comprise a direct measure of sweat amount (over time) based on skin conductivity measurements or actual sweat mass measurement, or a measure of a concentration of a sweat component (over time).

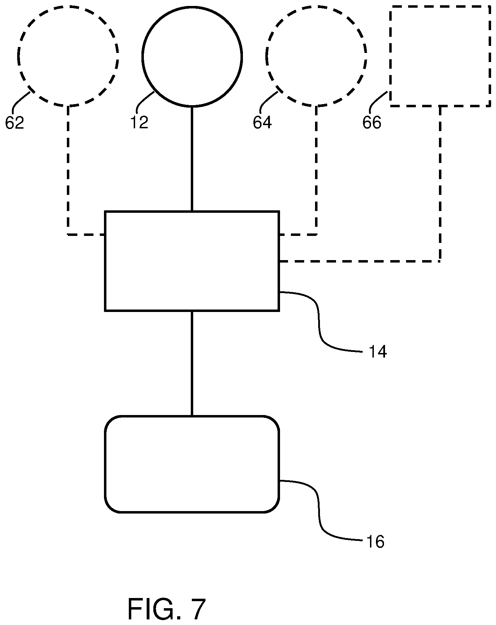

Figure 7 shows a second example of the system 10 for providing exercise advice according to the invention.

In analogy to the system of Figure 1, the system 10 comprises a sensor 12 for measuring a sweat parameter, a controller 14 and an output device 16.

The system of Figure 7 may further comprise a position and/or motion sensor 62. From the position and/or motion sensor can, for example, be an inertial sensor (e.g. an accelerometer or a gyroscope), a GPS sensor and combinations thereof. The motion sensor is connected to the controller 14 and the controller 14 is configured to derive the exercise load further taking account of the output of the position and/or motion sensor 20.

The use of inertial sensors and GPS sensors can help to contextualize the sweat rate measurements by classifying the activity state of the user and eliminate confounding environmental factors such as room humidity, wind/ventilation, and ambient temperature, which can influence sweat rate. If no exercise activity is detected, it could be assumed that any increase in the sweat rate is due to non-exercise related factors such as humidity, ambient temperature or emotionally or pathologically induced sweating. The activity state classification may be binary, i.e., indicate simply that the patient is exercising or not, or may be more sophisticated by classifying the specific type of exercise being performed by the patient, e.g., cycling vs. stair climbing. The latter classification may permit more specific advice/feedback to be given to the patient based on the type of exercise being performed and the measured sweat property. The sweat rate without activity can be taken as a baseline level, from which the changes during exercise should be determined.

Movement speed or frequency data obtained from an accelerometer can also be used to contextualize the sweat rate measurements in order to provide the right feedback to the patient in terms of exercise intensity. Indeed, any decrease in movement speed may cause rapid and sudden increase in the sweat rate because of lower cooling effect of moving body parts. Similarly, an increased intensity in terms of velocity and frequency may temporarily cause a drop in the sweat rate. This contextual information can be taken into account when analyzing temporal trends in the sweat rate and predict the optimal exercise load for a patient.

The system of Figure 7 can further comprise an environment sensor 64 for measuring at least one environmental condition connected to the controller 14. The controller is thereby configured to derive the exercise load further taking account of the output of the environment sensor 64.

The environmental sensor 64 is configured to detect environmental conditions such as ambient temperature, humidity or wind speed. Environmental conditions can have an impact on the sweat rate of a wearer. The controller 14 can increase the accuracy of the correlation between the sweat rate and the exercise level by taking account of environmental factors that influence the sweat rate for example by allowing for higher sweat rates in the case of elevated ambient temperatures or high humidity.

The system of Figure 7 may also comprise a communications interface 66 for receiving information from an external source to be used in deriving the exercise load.

The communications interface 24 can be any interface that is configured to receive data from an external source. For the convenience of the user, the use of wireless communication such as Bluetooth or Wi-Fi might be preferred.

Information that might impact the sweat rate of a wearer might include the ambient temperature or humidity in a room that can be obtained from wireless sensors such as for example a smart thermostat. Such information might also include weather forecasts or information from sensors in exercise equipment. The use of such information can further refine the correlation between the sweat rate and the exercise load for example by allowing higher sweat rates for higher temperatures and less ventilation.

In one possible implementation of the system of Figure 7, the controller 14 is further configured to determine a rate of change of the sweat rate and to derive the exercise load further taking account of the rate of change of the sweat rate.

Determining the rate of change in the sweat rate can help to discern between exercise related changes in the sweat rate which tend to happen over longer timescales of minutes and, for example, changes in the sweat rate due to emotional reasons which happen on a shorter timescale of seconds. In this case, the sensor 12 may be a GSR sensor. GSR sensors show a particularly rapid response to changes in the amount of sweat excreted and, hence are particularly suitable for identifying short timescale changes in the sweat rate that are not exercise related.

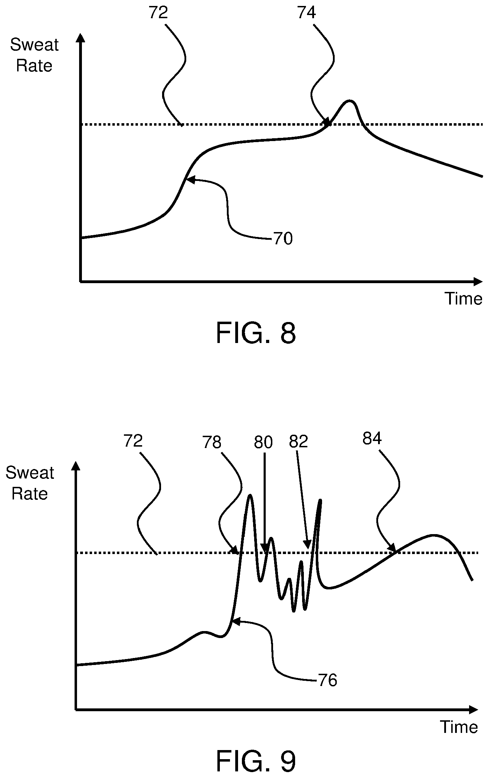

This is illustrated in Figures 7 and 8 where the sweat rates of two exercising subjects are compared. Figures 7 and 8 show a sweat rate over time, with a timescale of minutes.

In Figure 8, the sweat rate increases gradually as the exercise proceeds and the subject starts to sweat. In the case shown in Figure 8, the sweat rate 70 gradually approaches a defined threshold 72. As the sweat rate 70 exceeds the threshold 72 at the point indicated with reference numeral 74 a warning is correctly issued to moderate the exercise load.

In Figure 9, the sweat rate varies both with characteristic timescales of minutes and, for a portion of the measurement period, with timescales of seconds. In the case shown in Figure 9, two mechanisms of sweat production occur at the same time, the thermoregulation sweating (timescale minutes) and an emotionally based sweat production (timescale seconds).

Whilst the thermoregulation sweating correlates to exercise rate, the emotionally based sweating does not.

If the system does not take this factor into account, the system may issue four warnings during the measurement shown in Figure 9 when the sweat rate 76 exceeds the threshold 72 at points 78, 80, 82 and 84. However only the final warning at point 84 is related to an increased exercise level and the others are, in this case, false positives.

In order to order to improve the accuracy, it is desirable that the system is able to recognize and reject increases in the sweat rate above the threshold, which are the result of non-exercise related sweat production. This can be achieved by considering not only the absolute intensity of the sweat production but also the rate of increase in the sweat rate when triggering the system to issue warnings. If the threshold is exceeded but with a too high rate of change (timescale of order seconds) the higher sweat rate can be considered a false positive that will not trigger an alarm.

The above elimination of false positives can, for example be realized by processing the raw signal from the sensor 12 and extracting both amplitude and rate of change of sweat production and rejecting those alarms triggered by an increase in the sweat rate over too short a timescale. Another option is to introduce a hardware based low pass filter with cut-off around a few seconds to the sensor 12, for example integrated with the GSR sensing electronics, whereby the rapid sweat peaks are not transmitted to the controller and no false positives are generated.

The controller 14 in the system 10 of Figure 7 may be further configured to provide post-exercise advice.

The post-exercise advice may thereby be selected from advice on the intensity of the next exercise, advice on the duration of the next exercise, advice on the time to wait until the next exercise, sometimes also referred to as recovery time, and combinations thereof.

The sweat rate and/or the sweat volume can be used to forecast the maximum rate at which a patient should perform an upcoming exercise activity in order to remain within a safe limit (such as a heart rate range) based on the intensity of the current exercise activity being performed. For example, if the person suffering from heart failure is performing a moderate exercise activity resulting in a maximum measured sweat rate of 2.5 nl/min/gland and must perform another exercise activity of comparable or greater intensity, the system can advise the patient to lower the intensity of the current activity or to modify the intensity of the upcoming activity to ensure that they will always remain in a safe limit.

Such retrospective advice could also be based on the measured sweat after the exercise. When the sweat rate stays high for a long time (e.g. of the order of 1 hour or more) after the exercise, this hints to severe post-exercise hyperthermia and the advice should be to exercise less hard next time and to wait at least a day before starting the next exercise. On the other hand, when the sweat rate is back to a normal non-active baseline level already within 20 minutes after exercise, the advice could be to exercise harder next time and/or to even do one more exercise later on this day.

Post-exercise advice can further help a person suffering from heart failure plan and balance their exercise load to avoid further damage to the heart. This will increase the health benefit of exercise while at the same time help reducing the negative effects of over exercising.

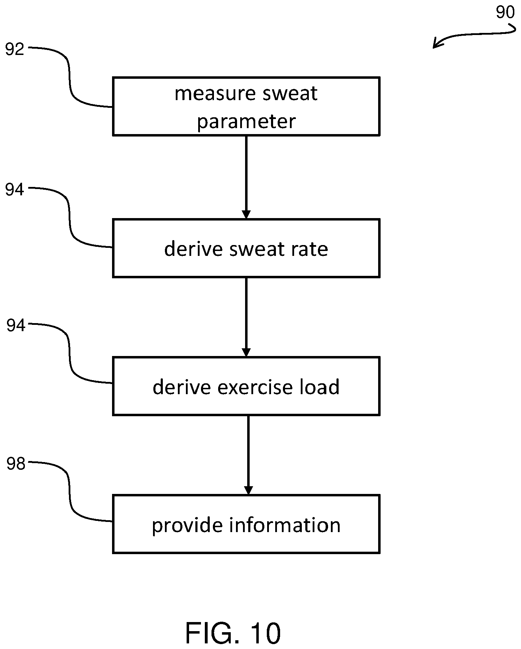

Figure 10 shows a flow diagram for a first example of a method 90 for providing exercise advice to a person.

In a first step 92 of the method 90, a sweat parameter of a person is measured. The sweat parameter can thereby be measured using any suitable sensor such as a GSR sensor, a calorimetric flow sensor, a microbalance or resonance based sensors. As explained above, the primary sweat parameter measurement may also be based on biomarker sensing.

In a second step 94, a sweat rate at which sweat is excreted by the person is derived from the sweat parameter measured in the first step.

In a third step 96 of the method 90, an exercise load is derived from the sweat rate determined in the second step.

In a fourth step 98 of the method 90, information is provided to the person relating to the derived exercise load. The information provided may be selected from advice to increase the intensity of the exercise, advice to decrease the intensity of the exercise, advice on the length of time the exercise should be continued for and a notification that the exercise needs to be stopped immediately.

The method 90 of Figure 10 can provide exercise advice in situations where other, conventional methods such as heart rate monitoring are not a suitable or reliable way to determine exercise intensity.

The method 90 is particularly useful for people suffering from heart failure as other, more conventional methods such as heart rate monitoring are not suitable to determine exercise intensity. The use of heart rate monitoring as a reliable tool for determining the exercise intensity can be precluded in people suffering from heart failure as they are often taking medication to moderate their heart rate. Furthermore, such patients can also show arrhythmias that might make heart rate monitoring unreliable.

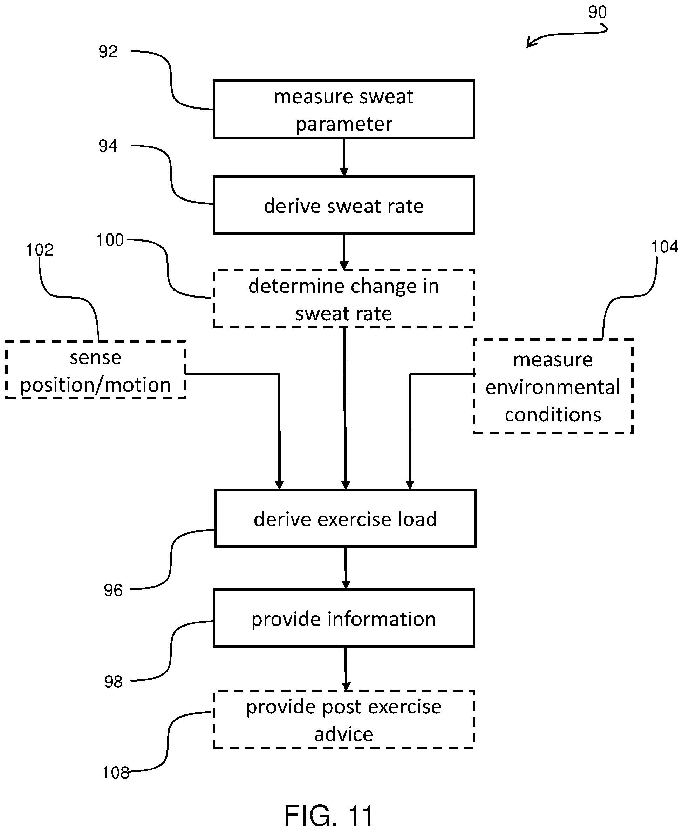

Figure 11 shows a flow diagram for a second example of a method 90 for providing exercise advice to a person.

In addition to the steps shown in Figure 3, the method 90 of Figure 11 can include further steps.

For example, the method 90 of Figure 11 can further comprise a step 100 of determining a rate of change in the sweat rate, and determining the exercise load further taking account of the rate of change.

Determining the rate of change in the sweat rate can help to discern between exercise related changes in the sweat rate which tend to happen over longer timescales of minutes and, for example, changes in the sweat rate due to emotional reasons which happen on a shorter timescale of seconds.

The method 90 of Figure 11 can further comprise a step 102 of measuring the concentration of at least one biomarker present in the sweat of the person and deriving the exercise load further taking account of the measured concentration.

The biomarker detected in this step may be a biomarker indicative of the exercise load. Suitable examples of biomarkers that are indicative of the exercise load include the concentration of sodium ions, potassium ions or lactate in the sweat. The determination of the exercise load in this case also takes account of the biomarker and its concentration in the sweat.

Biomarkers present in the sweat of a person can provide further information on the exercise load making the overall determination of the exercise level more reliable. Furthermore, these biomarkers can give additional indication of the level of health and fitness of a person.

In addition, the method 90 of Figure 11 can further comprise a step 104 of measuring environmental conditions around the person and/or a step 106 of sensing a position and/or motion of the person and deriving the exercise load further taking account of the measured environmental conditions and/or the movement of the person.

Sensing a position or motion of a person can provide further information on the nature of the exercise performed. For example, it may be possible to determine which form of exercise the wearer is performing, such a running or cycling. This helps to contextualize the data obtained. Furthermore, information on the nature of the exercise performed can be used to further refine the determination of the exercise load as well as identify external factors that might impact the sweat rate such the cooling effect of a slipstream.

Environmental conditions can have an impact on the sweat rate of a wearer. Measuring such environmental conditions can increase the accuracy of the correlation between the sweat rate and the exercise level by taking account of environmental factors that influence the sweat rate.

Environmental conditions can also be measured by sensors integrated in a device for providing exercise advice or external devices such as smart thermostats or hygrometers, which are already present in a room where the exercise is performed.

The method 90 of Figure 11 may further comprise a step 108 of providing post-exercise advice. The post-exercise advice may thereby be selected from advice on the intensity of the next exercise, advice on the duration of the next exercise, advice on the time to wait until the next exercise and combinations thereof.

Post-exercise advice can further help a person plan and balance their exercise load for example to avoid heart damage in a person suffering from heart failure.

It is to be understood that the advantages and ways of implementing the invention described above with respect to the systems of the invention also apply in analogy to the methods of the invention.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. Any reference signs in the claims should not be construed as limiting the scope. A single processor or other unit may fulfil the functions of several items recited in the claims. Measures recited in mutually different dependent claims may be advantageously combined. If the term "adapted to" is used in the claims or description, it is noted the term "adapted to" is intended to be equivalent to the term "configured to".

A computer program may be stored/distributed on a suitable medium, such as an optical storage medium or a solid-state medium supplied together with or as part of other hardware, but may also be distributed in other forms, such as via the Internet or other wired or wireless telecommunication systems.

## Claims

1. A system for providing exercise advice, the system comprising:
a wearable sensor (12) configured to measure a sweat parameter;
a controller (14); and
an output device (16),
wherein the controller (14) is configured to:
receive (92) the sweat parameter;
derive (94) from the sweat parameter a sweat rate at which sweat is excreted by a wearer,
derive (96) from the sweat rate an exercise load; and
control the output device (16) to provide (98) information to the wearer relating to the derived exercise load.

2. The system of claim 1, wherein the information provided to the wearer is selected from advice to increase the intensity of the exercise, advice to decrease the intensity of the exercise, advice on the length of time the exercise should be continued for and a notification that the exercise needs to be stopped immediately.

3. The system of claim 1 or 2, wherein:
the wearable sensor comprises a sensor configured to measure a total sweat volume or sweat rate; or
the wearable sensor comprises a sensor configured to measure at least one biomarker present in the sweat of the wearer; or
the wearable sensor comprises a sensor configured to measure a total sweat volume or sweat rate and the system further comprises a sensor for at least one biomarker present in the sweat of the wearer and the controller is configured to derive the exercise load taking account of the total sweat volume or sweat rate and the at least one biomarker.

4. The system of any one of claims 1 to 3, further comprising a position and/or motion sensor (62), wherein the controller (14) is configured to derive the exercise load further taking account of the output of the position and/or motion sensor (62).

5. The system of any of claims 1 to 4, further comprising an environment sensor (64) configured to measure at least one environmental condition, wherein the controller (14) is configured to derive the exercise load further taking account of the output of the environment sensor (64).

6. The system of any of claims 1 to 5, further comprising a communications interface (66) configured to receive information from an external source to be used in deriving the exercise load.

7. The system of any of claims 1 to 6, wherein the controller (14) is further configured to determine a rate of change of the sweat rate and to derive the exercise load further taking account of the rate of change of the sweat rate.

8. The system of any of claims 1 to 7, wherein the controller (14) is configured to provide post-exercise advice, selected from advice on the intensity of the next exercise, advice on the duration of the next exercise, advice on the time to wait until the next exercise and combinations thereof.

9. A method (90) for providing exercise advice to a person, the method comprising:
measuring (92) a sweat parameter of the person using a wearable sensor;
deriving (94) from the sweat parameter a sweat rate at which sweat is excreted by the person;
deriving (96) an exercise load from the sweat rate; and
providing (98) information to the person relating to the derived exercise load.

10. The method of claim 9, further comprising determining (100) a rate of change in the sweat rate, and determining the exercise load further taking account of the rate of change.

11. The method of claim 9 or 10, wherein the information relating to the derived exercise load is selected from advice to increase the intensity of the exercise, advice to decrease the intensity of the exercise, advice on the length of time the exercise should be continued for and a notification that the exercise needs to be stopped immediately.

12. The method of any one of claims 9 to 11, wherein measuring a sweat parameter comprises:
measuring a total sweat volume or sweat rate; or
sensing at least one biomarker present in the sweat of the wearer; or
measuring a total sweat volume or sweat rate and the method further comprises sensing at least one biomarker present in the sweat of the wearer, wherein deriving an exercise load takes account of the total sweat volume or sweat rate and the at least one biomarker.

13. The method of any of claims 9 to 12, further comprising measuring (104) environmental conditions around the person and/or sensing a position and/or motion of the person and deriving the exercise load further taking account of the measured environmental conditions and/or the movement of the person.

14. The method of any of claims 9 to 13, further comprising providing post-exercise advice, selected from advice on the intensity of the next exercise, advice on the duration of the next exercise, advice on the time to wait until the next exercise and combinations thereof.

15. A computer program comprising computer program code which is adapted, when said program is run on a computer, to implement the method (90) of any one of claims 9 to 14.
